# EUROPÄISCHE PATENTSCHRIFT

(11) **EP 1 174 125 B1**
(45) Veröffentlichungstag und Bekanntmachung des Hinweises auf die Patenterteilung: **23.11.2005**
(21) Anmeldenummer: 01117276.4
(22) Anmeldetag: 17.07.2001
(51) Int. Cl.: A61K 9/08, A61K 31/195

(54) **Verwendung von Taurin zur Herstellung einer intraokularen Infusionslösung**
Use of taurin in the manufacture of an intraocular infusion solution
Utilisation de taurine dans la fabrication d'une solution d'infusion intraoculaire

(30) Priorität: 21.07.2000 DE 10035620
(43) Veröffentlichungstag der Anmeldung: 23.01.2002
(73) Patentinhaber: *Acri.Tec Gesellschaft für ophthalmologische Produkte mbH, 16761 Hennigsdorf (DE)
(72) Erfinder: Krott, Ralf, Dr., 50937 Köln (DE); Bartz-Schmidt, Karl Ulrich, Prof. Dr. med., 50968 Köln (DE); Kreiner, Christine, Dr., 81545 München (DE)
(74) Vertreter: Nöth, Heinz

(56) Entgegenhaltungen:
- EP-A- 0 778 021
- WO-A-99/29315
- US-A- 4 837 021
- US-A- 5 856 329
- US-A- 5 877 211
- DATABASE MEDLINE [Online] US NATIONAL LIBRARY OF MEDICINE (NLM), BETHESDA, MD, US; BRIKMAN I V ET AL: "[A new enriched balanced irrigation solution for intraocular surgery (experimental study)]. Novyi obogashchennyi sbalansirovannyi irrigatsionnyi rastvor dlia vnutriglaznoi khirurgii (eksperimental'noe issledovanie)." retrieved from STN Database accession no. 90312796 XP002181860 & VESTNIK OFTALMOLOGII, (1990 MAR-APR) 106 (2) 47-50. ,
- DATABASE WPI Section Ch, Week 199708 Derwent Publications Ltd., London, GB; Class B05, AN 1997-083383 XP002181861 & JP 08 325143 A (TAISHO PHARM CO LTD), 10. Dezember 1996 (1996-12-10)
- DATABASE WPI Section Ch, Week 199703 Derwent Publications Ltd., London, GB; Class B05, AN 1997-034084 XP002181862 & WO 96 38149 A (TAISHO PHARM CO LTD), 5. Dezember 1996 (1996-12-05)

## Beschreibung

Die Erfindung betrifft eine intraokulare Infusionslösung für die Vitrektomie.

### [Stand der Technik]

Mit Vitrektomie bezeichnet man die operative Entfernung des Glaskörpers aus dem Auge. Hierbei werden die Instrumente durch Einschnitte in der Pars-plana des Ziliarkörpers des Auges eingeführt. Durch Zufuhr einer intraokularen wässrigen Lösung, insbesondere von physiologischer balancierter Salzlösung (BSS) ist es möglich, dass der Augapfel während der Operation seine normale Form beibehält. Während der Operation kann der Operateur über das Operationsmikroskop durch die Pupille des Auges die Instrumente im Augeninnern beobachten und extrem feine Manipulationen durchführen. Die Beleuchtung des Augeninnern erfolgt durch eine ebenfalls ins Auge eingeführte Sonde. Bei der Operation wird der Glaskörper mit speziellen Geräten ausgeschnitten und abgesaugt, wobei gleichzeitig das entfernte Glaskörpervolumen durch die intraokulare Infusions- oder Spüllösung ausgeglichen wird.

Hauptsächliche Indikationen zur Vitrektomie sind die fortgeschrittene diabetische Retinopathie, komplizierte Netzhautablösung mit Schrumpfung und Strangbildung sowie perforierende Verletzung mit Netzhautschrumpfung und Strangbildung.

Die Vitrektomie ist zwar ein sicherer und für den Patienten vorteilhafter Eingriff, es verbleiben jedoch Risiken, die durch die Methode des operativen Eingriffes verursacht sind. Diese iatrogene Gefahren ergeben sich aus der Toxizität des Lichts für die Retina bei der Beleuchtung des Augeninnern, insbesondere bei länger andauerndem operativen Eingriff. Ferner besteht die Gefahr mechanischer Zerstörungen an der Retina, insbesondere bei epi- und subretinalen Membranabschälen und/oder bei der Anwendung temporärer oder permanenter Endotamponade, insbesondere mit Flüssigkeiten, die schwerer als das Kammerwasser sind und Silikonöl.

### [Aufgabe der Erfindung]

Aufgabe der Erfindung ist es, eine intraokulare Infusionslösung für die Vitrektomie zu schaffen, bei welcher aus dem operativen Eingriff resultierende iatrogene Risiken verringert oder beseitigt sind.

Diese Aufgabe wird erfindungsgemäß durch die Merkmale des Patentanspruches 1 gelöst.

Bei der Erfindung wird der intraokularen wässrigen Infusionslösung, welche als Spüllösung bei der Vitrektomie verwendet wird, ein neuroprotektives Agens, nämlich Taurin, zugesetzt. Taurin (2-Aminoethansulfonsäure) entsteht im Organismus aus Cystein und ist in der Galle an Cholsäure als Amid verbunden. Taurin wurde erstmals aus Ochsengalle hergestellt. Beim Kochen von Ochsengalle mit Säure wird Taurin aus der Taurocholsäure abgespalten. Taurin kommt als neuroinhibierende Aminosäure zur Behandlung von Anfällen zum Einsatz ([1]-[3]). Ferner wird es als Membranstabilisator und zur Verringerung von Herz-Rythmus-Störungen eingesetzt [4]. Ferner haben Tierversuche gezeigt, dass Taurinmangel zur Netzhautdegeneration führen kann ([5] und [6]). Untersuchungen haben ferner gezeigt, dass Taurin das Wachstum und die Differenziation unterschiedlicher Zelltypen, wie Pigmentepithelzellen der menschlichen Retina und menschliche mikrovaskuläre Endothelzellen nicht beeinflusst ([7] und [8]).

Demgegenüber wird bei der Erfindung Taurin als neuroprotektives Agens in einer intraokularen Perfussionslösung eingesetzt, wodurch iatrogene Schädigungen der Retina bei der Vitrektomie minimiert oder verhindert werden.

Bestätigt wird dies durch Versuche, welche mit Genistein an Rinderretina durchgeführt wurden. Genistein ist ein bekannter Inhibitor von Tyronsinkinase im Protein. Elektroretinographie(ERG)-Untersuchungen zeigten, dass die toxischen Wirkungen von Genistein auf die Retina verringert werden, wenn Taurin zugefügt wird. Die neuroprotektive Wirkung wird in einem Bereich von 325 µg/ml bis 425 µg/ml Taurin in der Spüllösung erreicht. Bevorzugt wird ein Taurin-Anteil von 375,3 µg/ml in der intraokularen Spüllösung eingesetzt.

### [Beispiele]

Anhand der Figuren wird die Erfindung noch näher erläutert.

Es zeigt
- Fig. 1 bis 4: Elektroretinogramme bei der Behandlung von Rinderretina mit Genistein mit unterschiedlichen Konzentrationen mit und ohne Zugabe von Taurin; und
- Fig. 5: schematisch den operativen Vorgang der Vitrektomie, bei welcher ein Ausführungsbeispiel der Spüllösung mit Taurin zum Einsatz kommt.

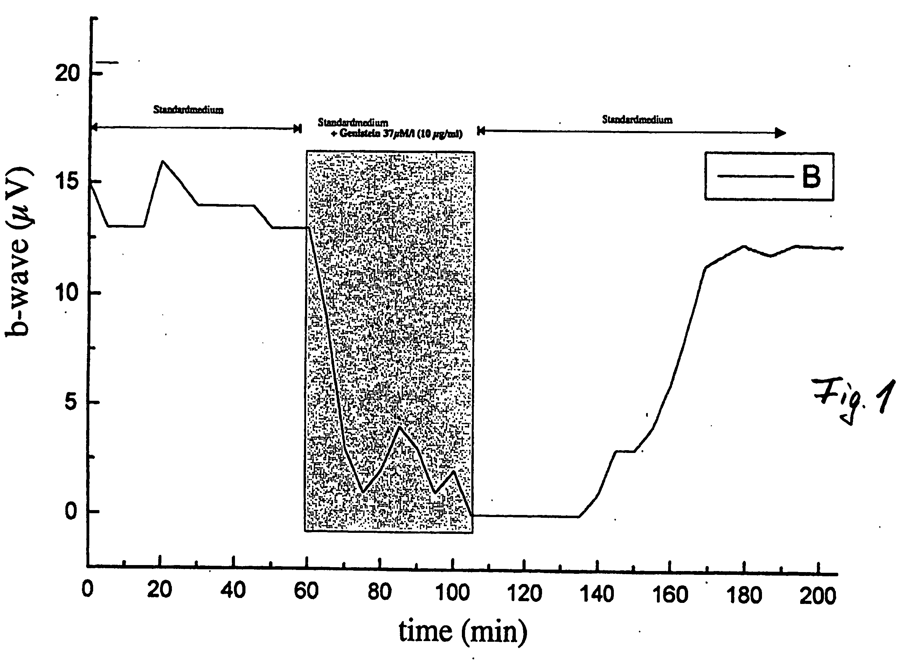

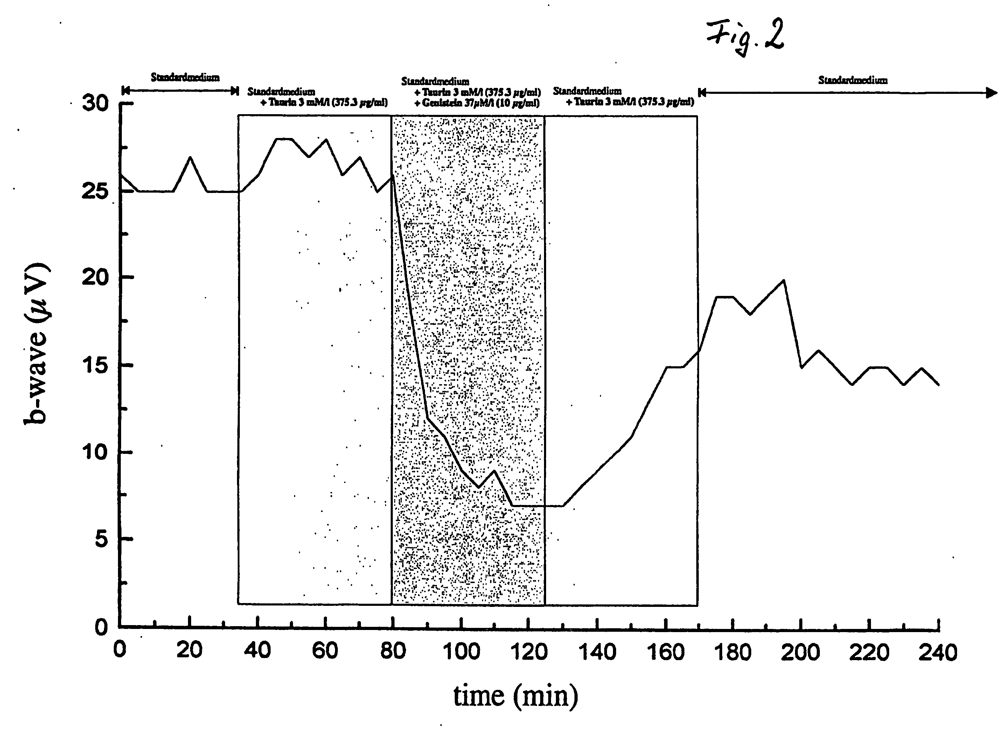

Die Fig. 1 zeigt eine b-Welle in Abhängigkeit von der Zeit für eine Behandlung einer Rindernetzhaut mit Genistein niedriger Konzentration (10 µg/ml). Aus der Fig. 1 ist zu ersehen, dass die b-Welle des Elektroretinogramms (ERG) nach 45 Min. vollständig erloschen ist. Bei Hinzugabe von Taurin in einem Anteil von 375,3 µg/ml (Fig. 2) wurde das vollständige Erlöschen der b-Welle vermieden.

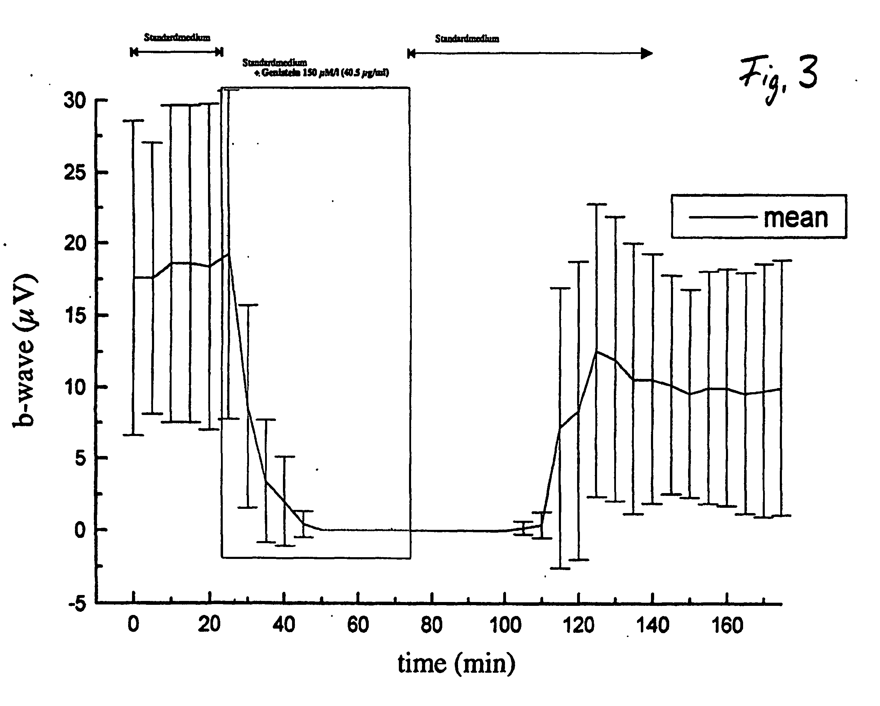

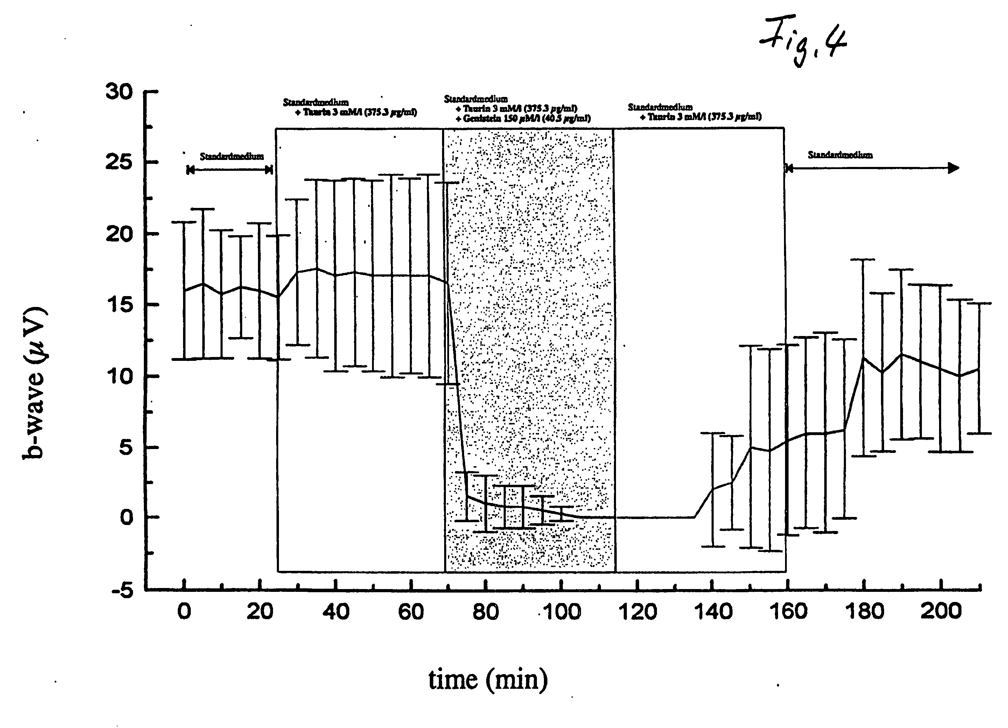

Die Fig. 3 zeigt die Messergebnisse bei Anwendung einer höheren Konzentration 40,5 µg/ml im Standardmedium, wobei nach 20 Min. Spülung die b-Welle bereits vollständig ausgelöscht war. Bei der Zugabe von Taurin in einem Anteil von 375,3 µg/ml ergab sich erst nach 35 Min. eine vollständige Auslöschung der b-Welle (Fig. 4). Gemäß Fig. 4 ergab sich bei der Behandlung von Genistein allein eine Erholung der b-Welle nach 50 Min. (Fig. 3) und bei der Zugabe von Taurin ergab sich eine Erholung der b-Welle bereits nach 30 Min. (Fig. 4).

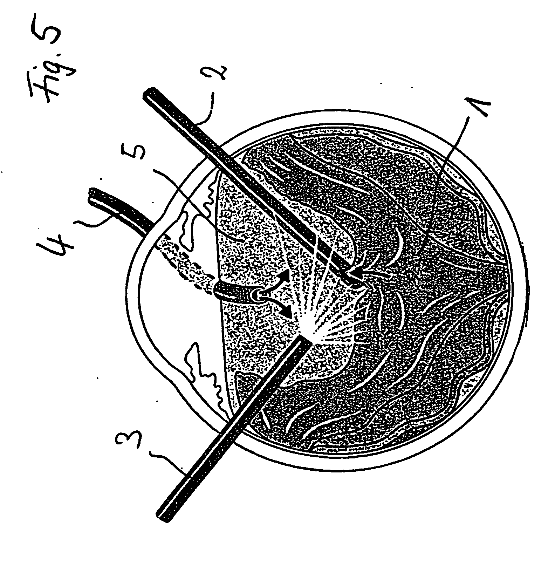

In der Fig. 5 ist schematisch die Technik der Vitrektomie dargestellt. Über der Pars-plana des Augenziliarkörpers werden in einem oberen Umfangsbereichs des Augapfels drei Öffnungen angelegt. Über die in der Fig. 5 rechte Öffnung wird ein Glaskörperschneidgerät 2 in den Glaskörperraum eingeführt. Über die in der Fig. 5 mittlere Öffnung wird eine Infusionskanüle 5 in den Glaskörperraum eingeführt und über die in der Fig. 5 linke Öffnung ist eine Lichtquelle 3, welche einen Lichtleiter mit Glasfaseroptik in bekannter Weise enthalten kann, in den Glaskörperraum eingeführt. Im Bereich des distalen Endes besitzt das Glaskörperschneidgerät 2 eine Schneidkante mit angeschlossenem Saugrohr, durch welches aus dem Glaskörperraum zu entfernender Glaskörper 1 abgesaugt wird. Das entfernte Glaskörpervolumen wird gleichzeitig mit Hilfe einer balancierten physiologischen Salzlösung 5, welche Taurin enthält, ausgeglichen. Diese physiologische Infusionslösung wird über die Infusionskanüle 4 in den Glaskörperraum eingeleitet. Mit Hilfe der Lichtquelle 3 wird der Glaskörperraum ausgeleuchtet.

Durch die Zugabe von Taurin als Neuroprotektionsagens zur Infusionslösung 5 werden iatrogene Verletzungen der Netzhaut, welche aus der Ausleuchtung des Glaskörperraums oder aus mechanischen Beeinflussungen der Retina beim Abziehen von epi- und subretinalen Membranen und/oder aus nach der Vitrektomie durchgeführten temporären oder langzeitlichen Endotamponaden resultieren können, minimiert oder verhindert.

### Literatur

- [1]: van Gelder NM, Koyama I et al. Taurine treatment of spontaneous chronic epilepsy in a cat. Epilepsia 1977; 18(1):45-54
- [2]: Pisarenko OI. Mechanisms of myocardial protection by amino acids: Facts and hypotheses. Clin Exp Pharmacol Physiol 1996; 23(8) : 627-33
- [3]: Fujita T. Sato Y. Hypotensive effect of taurine. Possible involment of the sympathetic nervous system and endogenous opiates. J. Clin Invest 1988;82(3): 993.97
- [4]: Azuma J et al. Beneficial effect of taurine on congestive heart failure induced by chronic aortic regurgitation in rabbits. Res Commun Chem Pathol Pharmacol 1984; 45(2): 261-70
- [5]: Gaby AR, Wright JV. Nutritional factors in degenerative eye disorders: Cataract and macular degeneration. J Adv Med. 1993; 61(1): 27-40
- [6]: Hayes KC, Carey RE et al. Retinal degeneration associated with taurine deficiency in the cat. Science 1975; 188(4191): 949-51
- [7]: Krott R. Kociok N., Lüke M., Lüke C., Esser P., Bartz-Schmidt KU. Taurine does not interfere with the growth control of genistein treated human microvascular endothelial cells. Invest Ophthalmol Vis Sci 2000; 41; 633
- [8]: Lüke M., Krott R. Kociok N., Lüke C., Esser P., Bartz-Schmidt KU. Taurine does not interfere with the growth control of genistein treated human retinal pigment epithelium cells. Invest Opthalmol Vis Sci 2000; 41: 604

### [Bezugszeichenliste]

- 1: Glaskörper
- 2: Glaskörperschneidgerät
- 3: Lichtquelle
- 4: Infusionskanüle
- 5: Taurin-haltige Infusionslösung

## Patentansprüche

1. Verwendung von Taurin zur Herstellung einer Infusionslösung zum Ausgleich von bei der Vitrektomie entferntem Glaskörpervolumen mit einem Anteil des Taurins von 325 µg/ml bis 425 µg/ml in der Lösung als neuroprotektives Agens.

2. Verwendung nach Anspruch 1,
wobei Taurin in einem Anteil von etwa 375,3 µg/ml in der Lösung vorliegt.

## Claims

1. Use of taurin for the production of an infusion solution for the compensation of vitreous humour volume removed in vitrectomy with a proportion of taurin of 325 µg/ml to 425 µg/ml in the solution as a neuroprotective agent.

2. Use according to claim 1 wherein taurin is present in a proportion of about 375.3 µg/ml in the solution.

## Revendications

1. Utilisation de taurine pour la préparation d'une solution d'injection pour compenser le volume de corps vitré retiré lors de la vitrectomie contenant une concentration de taurine de 325 µg/ml à 425 µg/ml dans la solution en tant qu'agent neuro-protecteur.

2. Utilisation suivant la revendication 1, dans laquelle la taurine est présente en une concentration d'environ 375,3 µg/ml dans la solution.
